(19) Europäisches Patentamt
European Patent Office
Office européen des brevets

(11) EP 1 272 491 B1

(12) EUROPÄISCHE PATENTSCHRIFT

(45) Veröffentlichungstag und Bekanntmachung des Hinweises auf die Patenterteilung:
**14.01.2004 Patentblatt 2004/03**

(21) Anmeldenummer: **01915325.3**

(22) Anmeldetag: **10.03.2001**

(51) Int Cl.[7]: **C07D 471/06**, C07D 491/06, C07D 493/06, C09B 5/62, C09B 5/00
// (C07D471/06, 221:00), C07D221:00,(C07D491/06, 311:00), C07D221:00,(C07D493/06, 311:00), C07D311:00

(86) Internationale Anmeldenummer:
**PCT/EP2001/002701**

(87) Internationale Veröffentlichungsnummer:
**WO 2001/068650 (20.09.2001 Gazette 2001/38)**

# (54) KRISTALLISATIONSMODIFIKATOREN AUF DER BASIS VON PERYLENDERIVATEN

PERYLENE-DERIVATIVE BASED CRYSTALLIZATION MODIFIERS

AGENTS DE MODIFICATION DE CRISTALLISATION SE BASANT SUR DES DERIVES DE PERYLENE

(84) Benannte Vertragsstaaten:
**DE FR GB IT**

(30) Priorität: **17.03.2000 DE 10013188**

(43) Veröffentlichungstag der Anmeldung:
**08.01.2003 Patentblatt 2003/02**

(73) Patentinhaber: **BASF AKTIENGESELLSCHAFT**
**67056 Ludwigshafen (DE)**

(72) Erfinder:
- **HACKMANN, Claus**
**67281 Kirchheim (DE)**
- **GÜNTHERT, Paul**
**67105 Schifferstadt (DE)**
- **DOTTER, Anton**
**68766 Hockenheim (DE)**
- **BLASCHKA, Peter**
**67069 Ludwigshafen (DE)**

(56) Entgegenhaltungen:
**EP-A- 0 302 973**
**WO-A-91/02034**
**US-A- 4 431 806**
**US-A- 5 248 774**
**US-A- 5 264 034**
**US-A- 5 271 759**
**US-A- 5 466 907**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft Perylenderivate der allgemeinen Formel I

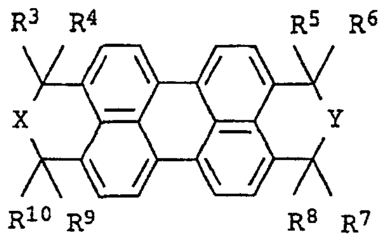

I

in der die Variablen folgende Bedeutung haben:

X und Y unabhängig voneinander Sauerstoff, $-NR^1$ oder $-NR^2$;

$R^1$ und $R^2$ unabhängig voneinander Wasserstoff, $C_1$-$C_{18}$-Alkyl, $C_5$-$C_{-7}$-Cycloalkyl, Aryl oder $C_1$-$C_6$-Alkoxy;

$R^3$ bis $R^{10}$ unabhängig voneinander Wasserstoff, Hydroxy oder Aryl, wobei gemeinsam an ein Kohlenstoffatom gebundene Reste auch =O oder $=CHR^{11}$ bedeuten können;

$R^{11}$ Wasserstoff oder $C_1$-$C_3$-Alkyl,

mit der Maßgabe, daß das Perylenderivat I mindestens eine bis maximal drei Carbonylgruppen pro Molekül enthält.

Außerdem betrifft diese Erfindung die Verwendung der Perylenderivate I als Kristallisationsmodifikatoren für organische Pigmente sowie Pigmentzubereitungen, welche die Perylenderivate I enthalten.

Perylenpigmente sind bereits seit langem bekannt. Sie zeichnen sich durch ihre hohen Farbstärken und Licht- und Wetterechtheiten aus und haben große Bedeutung für die Lack- und Kunststoffeinfärbung. Als Beispiele für besonders interessante Vertreter dieser Pigmentklasse seien N,N'-Dimethylperylen-3,4,9,10-tetracarbonsäurediimid (C. I. Pigment Red 179), N,N'-Bis(4-phenylazophenyl)perylen-3,4,9,10-tetracarbonsäurediimid (C.I. Pigment Red 178) und N,N'-Bis(3,5-dimethylphenyl) perylen-3,4,9,10-tetracarbonsäurediimid (C.I. Pigment Red 149) genannt.

Bei der Synthese dieser Pigmente fallen jedoch Rohpigmente mit anwendungstechnisch ungünstiger Teilchenform und TeilchengröBe an, die einer Nachbehandlung, z.B., wie in der DE-A-21 53 087 beschrieben, einer Salzmahlung oder -knetung bedürfen, um in eine für die Anwendung geeignete Pigmentform überführt zu werden.

Weiterhin ist auch der Zusatz von Substanzen, welche die Kristallisation des Rohpigments beeinflussen und die Bildung transparenter Pigmente fördern sollen, bekannt. So wird das Rohpigment in der EP-A-807 668 zu diesem Zweck einer Säurequellung in Gegenwart von Anthanthron-, Chinacridon- und Flavanthronpigmenten unterzogen. In der WO-A-91/02034 sind Dispergatoren auf Basis von an einem oder beiden Imidstickstoffatomen durch Alkylen- bzw. Arylensulfonsäuregruppen substituierten Perylen-3,4,9,10-tetracarbonsäuremonoanhydridmonoimiden oder -diimiden beschrieben, welche die Pigmentoberfläche belegen. Diese Pigmentzubereitungen weisen jedoch durch das zugesetzte Pigment veränderte Koloristik auf oder sind nur eingeschränkt einsetzbar.

Der Erfindung lag daher die Aufgabe zugrunde, Kristallisationsmodifikatoren bereitzustellen, mit deren Hilfe transparente Perylenpigmente erhalten werden können, die hervorragende anwendungstechnische und koloristische Eigenschaften aufweisen.

Demgemäß wurden die Perylenderivate der eingangs definierten Formel I und deren Verwendung als Kristallisationsmodifikatoren für organische Pigmente gefunden.

Weiterhin wurden Pigmentzubereitungen gefunden, welche

A) mindestens ein organisches Pigment aus der Klasse der Perylenpigmente und

B) mindestens ein Perylenderivat der Formel I sowie gewünschtenfalls

C) mindestens ein Kolophoniumharz

enthalten.

Alle in Formel I auftretenden Alkylreste können sowohl geradkettig als verzweigt sein. Sie enthalten bis zu 18 C-Atome, wobei $C_1$-$C_4$-Alkylreste, insbesondere Methyl, bevorzugt sind. Im einzelnen seien beispielhaft genannt: Methyl, Ethyl, Propyl, Isopropyl, Butyl, Isobutyl, sec.-Butyl, tert.-Butyl, Pentyl, Isopentyl, Neopentyl, tert.-Pentyl, Hexyl, 2-Methylpentyl, Heptyl, 1-Ethylpentyl, Octyl, 2-Ethylhexyl, Isooctyl, Nonyl, Isononyl, Decyl, Isodecyl, Undecyl, Dodecyl, Tridecyl, Isotridecyl, Tetradecyl, Pentadecyl, Hexadecyl, Heptadecyl und Octadecyl (die obigen Bezeichnungen Isooctyl, Isononyl, Isodecyl und Isotridecyl sind Trivialbezeichnungen und stammen von den nach der Oxosynthese erhaltenen Alkoholen).

Geeignete Cycloalkylreste sind Cyclopentyl, Cyclohexyl und Cycloheptyl.

Als Beispiele für geeignete Arylreste sind neben α- und β-Naphthyl vor allem Phenyl und substituiertes Phenyl wie 4-Phenylazophenyl und alkylsubstituiertes Phenyl, z.B. 3,5-Dimethylphenyl, die neben $C_1$-$C_9$-Alkyl bevorzugte Bedeutungen für $R^1$ und $R^2$ sind, zu nennen.

Die in Formel I auftretenden Alkoxyreste können ebenfalls geradkettig oder verzweigt sein. Beispielhaft aufgeführt seien Methoxy, Ethoxy, Propoxy, Isopropoxy, Butoxy, Isobutoxy, sec.-Butoxy, tert.-Butoxy, Pentoxy, Isopentoxy, Neopentoxy, tert.-Pentoxy und Hexoxy.

Bevorzugt sind Perylenderivate der Formel I, in der $R^5$ und $R^6$ sowie $R^7$ und $R^8$ jeweils zusammen =O bedeuten, d.h., Perylenderivate, die mindestens zwei Carbonylgruppen enthalten.

Besonders bevorzugt sind Perylenderivate der Formel I, in der $R^3$, $R^4$, $R^9$ und $R^{10}$ Wasserstoff oder Hydroxy bedeuten, wobei $R^3$ und $R^4$ oder $R^9$ und $R^{10}$ zusammen auch =O bedeuten können, und $R^5$ und $R^6$ sowie $R^7$ und $R^8$ jeweils zusammen =O bedeuten.

Vorzugsweise bedeutet dabei nur jeweils einer der beiden an ein Kohlenstoffatom gebundenen Reste Hydroxy.

Beispiele für ganz besonders bevorzugte Perylenderivate sind:

| Perylen-derivat | X | Y | $R^3$, $R^4$ | $R^5$, $R^6$ | $R^7$, $R^8$ | $R^9$, $R^{10}$ |
|---|---|---|---|---|---|---|
| Ia | O | O | OH, H | =O | =O | H, H |
| Ib | O | O | =O | =O | =O | H, H |
| Ic | O | O | H, H | =O | =O | H, H |
| Id | $NCH_3$ | $NCH_3$ | =O | =O | =O | H, H |
| Ie | $NCH_3$ | $NCH_3$ | OH, H | =O | =O | =O |
| If | O | $NCH_3$ | H, H | =O | =O | H, H |
| Ig | $NCH_3$ | O | OH, H | =O | =O | =O |
| Ih | $NC_2H_5$ | O | OH, H | =O | =O | =O |
| Ik | $NCH_3$ | $NCH_3$ | H, H | =O | =O | H, H |
| Im | O | O | $=CH_2$ | =O | =O | =O |
| In | O | $NCH_3$ | $=CH_2$ | =O | =O | =O |

Insbesondere hervorzuheben sind dabei die Perylenderivate Ia, Ib, Ie und Ik.

Zur Herstellung der erfindungsgemäßen Perylenderivate I kann von Perylen-3,4,9,10-tetracarbonsäureanhydrid, N-substituierten oder nicht substituierten Perylen-3,4,9,10-tetracarbonsäuremonoanhydridmonoimiden oder N, N'-substituierten oder nicht substituierten Perylen-3,4,9,10-tetracarbonsäurediimiden als Edukten ausgegangen werden.

Durch Reduktion dieser Edukte können die besonders bevorzugten Perylenderivate der Formel I, in der $R^3$, $R^4$, $R^9$ und $R^{10}$ Wasserstoff oder Hydroxy bedeuten, wobei $R^3$ und $R^4$ oder $R^9$ und $R^{10}$ zusammen auch =O bedeuten können, und $R^5$ und $R^6$ sowie $R^7$ und $R^8$ jeweils zusammen =O bedeuten, vorteilhaft erhalten werden.

Als Reduktionsmittel werden dabei vorzugsweise komplexe Hydride der allgemeinen Formel II

$$M^1(M^2H_mR'_n)_p \qquad \text{II}$$

eingesetzt, in der die Variablen folgende Bedeutung haben:

$M^1$ ein p-wertiges Metallkation wie Lithium, Natrium, Magnesium oder Aluminium;

$M^2$ Bor oder Aluminium, wobei gilt: $M^1$ # $M^2$;

R' Alkyl oder Alkoxy oder, wenn $M^2$ Bor ist, das Boratom im Ring enthaltendes Cycloalkyl;

m 1 bis 4;
n 0 bis 3, wobei gilt: m + n = 4;
p 1 bis 3.

Als geeignete Hydride II seien beispielsweise genannt: $LiBH_4$, $NaBH_4$, $LiAlH_4$, $NaAlH_4$, $Mg(BH_4)_2$, A1 $(BH_4)_3$, $LiAlH[OC[CH_3)_3]_3$, $NaAlH_2(C_2H_5)_2$ und $NaAlH_2(OC_2H_4OCH_3)_2$. Dabei sind Natriumborhydrid und Lithiumaluminiumhydrid bevorzugt.
Die Umsetzung mit dem Hydrid II kann in wäßriger Phase oder in einem organischen Lösungsmittel vorgenommen werden.
Bei Verwendung von Natriumborhydrid ist Wasser oder ein dipolaraprotisches Lösungsmittel, das nicht mit dem Hydrid reagiert, z.B. Dimethylsulfoxid, N-Methylpyrrolidon oder Dimethylacetamid, als Reaktionsmedium besonders geeignet.
Im Fall von Lithiumaluminiumhydrid ist ein unpolar-aprotisches Lösungsmittel, z.B. Tetrahydrofuran oder Diethylether, vorzuziehen.
Durch gezielte Auswahl der Reaktionsbedingungen (Menge an Hydrid II, Reaktionstemperatur und Reaktionsdauer) kann die Reduktion problemlos gesteuert werden.
Dies soll am Beispiel der erfindungsgemäßen Perylenderivate Ia bis Ic veranschaulicht werden, die vorteilhaft auf folgende Weise ausgehend von Perylen-3,4,9,10-tetracarbonsäuredianhydrid hergestellt werden können:
Da im Fall dieser Perylenderivate I nur eine Seite des Moleküls reduziert werden soll, empfiehlt es sich, das Perylen-3,4,9,10-tetracarbonsäuredianhydrid zunächst in das Monokaliumsalz zu überführen, das außerdem auch eine höhere Löslichkeit aufweist. Dieses kann nach Zwischenisolierung oder direkt unter den nachstehenden Bedingungen mit dem Hydrid II (z.B. Natriumborhydrid, Menge bezogen auf 1 g Perylen-3,4,9,10-tetracarbonsäuredianhydrid) umgesetzt werden:

0,9 bis 1,1 g Hydrid II, 10 bis 15°C, 45 bis 55 h → Perylenderivat Ib;
1,8 bis 2,2 g Hydrid II, 50 bis 80°C, 2 bis 4 h → Perylenderivat Ia;
3,5 bis 4,0 g Hydrid II, 90 bis 95°C, 4 bis 7 h → Perylenderivat Ic.

Nach erfolgter Umsetzung wird das Kaliumsalz durch Zugabe von Säure wieder in die Anhydridform überführt.
Selbstverständlich kann das Perylen-3,4,9,10-tetracarbonsäuredianhydrid auch direkt hydriert werden. Aufgrund der geringeren Löslichkeit des Anhydrids sind jedoch höhere Reaktionstemperaturen und Reaktionszeiten und größere Mengen Hydrid II er-5 forderlich, weshalb die Perylenderivate I auf diese Weise of tmals nicht gezielt hergestellt werden können, sondern Gemische verschiedener Reduktionsprodukte anfallen.
Durch Umsetzung mit dem gewünschten, vorzugsweise primären, Amin 0 (Alkyl-, Cycloalkyl-, Arylamin) können die erfindungsgemäßen Perylenderivate Ib und Ic anschließend in die entsprechenden Imide Id und If überführt werden. Im Fall von Ia entsteht auf diesem Weg üblicherweise das Imid Ie im Gemisch mit dem nicht reduzierten Pigment. Als Reaktionsmedium kann Wasser oder ein 5 Alkohol, z.B. Ethanol, oder deren Mischungen verwendet werden, wobei Wasser bevorzugt ist. Die Reaktionstemperaturen betragen im allgemeinen 60 bis 90°C, die Umsetzung kann unter Druck oder drucklos durchgeführt werden.
Als weitere Beispiele für erfindungsgemäße Perylenderivate der Formel I, in der X $-NR^1$ und Y Sauerstoff bedeutet, seien die Perylenderivate Ig und Ih genannt, die analog aus dem N-alkylsubstituierten Perylen-3,4,9,10-tetracarbonsäuremonoanhydridmonoimid über das Kaliumsalz unter Verwendung von 0,7 bis 1 g Natriumbor-5 hydrid je g Monoanhydridmonoimid bei 55 bis 60°C in 40 bis 55 h hergestellt werden können.
Weiterhin sind erfindungsgemäße Perylenderivate der Formel I, in der X und Y $-NR^1$ bedeuten, durch Umsetzung des entsprechenden ) N,N'-disubstituierten Perylen-3,4,9,10-tetracarbonsäurediimids mit dem Hydrid II unter gleichzeitiger Mahlung zugänglich. Da für diese Reaktion Temperaturen von etwa 100 bis 120°C erforderlich sind, wird zweckmäßigerweise ein hochsiedendes organisches Lösungsmittel, z.B. Dimethylsulfoxid, als Reaktionsmedium verwendet. Die Umsetzung dauert üblicherweise 3 bis 6 h. In Abhängigkeit von der gewählten Hydridmenge (0,8 bis 1,1 g bzw. 1,7 bis 2 g je g Perylimid) können die Perylenderivate Ie bzw. Ik hergestellt werden.
Grundsätzlich besteht auch die Möglichkeit, die Herstellung der Perylenderivate I mit der Synthese des Perylenpigments zu kombinieren, indem man das bei der Synthese anfallende Reaktionsgemisch mit einem Unterschuß an Hydrid II anreduziert. Auf diese Weise werden die Perylenderivate I zwar nicht gezielt erzeugt, es werden jedoch ebenfalls transparente Pigmentzubereitungen mit hervorragenden Anwendungseigenschaften erhalten.
Schließlich können durch Umsetzung insbesondere von Perylen-3,4,9,10-tetracarbonsäuredianhydrid mit

Metallorganylen auch Perylenderivate I erhalten werden, die anstelle eines Carbonylsauerstoff atoms eine $=CHR^{11}$-Gruppierung enthalten.

Besonders geeignet sind hierfür Metallorganyle der allgemeinen Formel III

$$(R'')_qM^3 \qquad III$$

in der die Variablen folgende Bedeutung haben:

- $M^3$ HalMg, Li, Na oder $Li_2CuCN$;
- Hal Chlor, Brom oder Iod;
- R " Alkyl, Cycloalkyl, Aryl oder Alkoxy;
- q 1 oder, wenn $M^3$ $Li_2CuCN$ ist, 2.

Als Beispiele für besonders geeignete Metallorganyle III seien Lithiumorganyle, wie Methyllithium und Butyllithium, und vor allem die Grignardverbindungen R"MgHal, z.B. $CH_3MgCl$, genannt, mit denen eine $=CH_2$-Gruppierung bzw. eine $=C_4H_8$-Gruppierung in das Perylenderivat I eingeführt werden kann.

Die Umsetzung mit den Metallorganylen III wird üblicherweise in Gegenwart eines unpolar-aprotischen Lösungsmittels, z.B. Tetrahydrofuran, durchgeführt.

Als Beispiel für diesen Reaktionstyp sei die Herstellung des Perylenderivats In durch Umsetzung von Perylen-3,4,9,10-tetracarbonsäuredianhydrid mit Methylmagnesiumchlorid aufgeführt. Durch anschließende Umsetzung mit einem primären Amin kann das entsprechende Monoimid I (z.B. In) hergestellt werden.

Analog können durch Umsetzung mit Metallorganylen der Formel III'

$$R'''M^4 \qquad III'$$

in der R''' Aryl und $M^4$ Lithium oder Natrium bedeutet, Arylgruppen in die erfindungsgemäßen Perylenderivate I eingeführt werden. Als besonders geeignetes Metallorganyl III' sei hier Phenyllithium genannt.

Die erfindungsgemäßen Perylenderivate I eignen sich hervorragend als Kristallisationsmodifikatoren für organische Pigmente, insbesondere für Perylenpigmente.

Die Perylenderivate I können dazu bereits bei der Pigmentsynthese zugesetzt werden. Vorzugsweise kommen die Perylenderivate I jedoch erst bei der Mahlung des Rohpigments zum Einsatz. In der Regel wird die Mahlung in Gegenwart von 0,02 bis 5 Gew.-%, vorzugsweise 0,2 bis 2 Gew.-% Perylenderivat I, bezogen auf die resultierende Pigmentzubereitung, durchgeführt. Geeignet ist sowohl eine Trockenmahlung, an die üblicherweise noch eine Quellung angeschlossen wird, als auch eine Naßmahlung in üblicherweise für eine Quellung verwendeten Lösungsmitteln, insbesondere in wäßrigem Medium.

Um den Mahlvorgang zu erleichtern, empfiehlt es sich, ein Harz zuzusetzen. Bevorzugt sind dabei Kolophoniumharze, wie Kolophonium selbst und seine allgemein bekannten Derivate, z.B. dimerisiertes, polymerisiertes und hydriertes Kolophonium und seine Umsetzungsprodukte mit Malein- und Fumarsäureanhydrid. Es können bis zu 18 Gew.-%, vorzugsweise 8 bis 15 Gew.-% Harz, bezogen auf die resultierende Pigmentzubereitung, zugesetzt werden.

Selbstverständlich können weitere für Pigmentzubereitungen übliche Zusatzstoffe, wie oberflächenaktive Mittel, zum Einsatz kommen. Geeignete Mengen für diese Zusatzstoffe betragen im allgemeinen 0 bis 5 Gew.-%.

Die Trockenmahlung kann in einer Kugelmühle, Planetenmühle oder Strahlmühle durchgeführt werden. Für eine Naßmahlung eignen sich insbesondere eine Kugelmühle oder Rührwerkskugelmühle, die vorzugsweise mit 100 bis 2000 Upm betrieben wird. Geeignete Mahlkörper sind z.B. Silicium/Aluminium/Zirkoniumoxidperlen (SAZ), Glasperlen, Achatkugeln oder Sandkörner, die Durchmesser im Bereich von etwa 1 cm oder von 0,3 bis 30 mm aufweisen können.

Üblicherweise wird solange gemahlen, bis eine mittlere Pigmentteilchengröße ($d_{50}$-Wert) von etwa 30 bis 100 nm erreicht ist. Die Trockenmahlung dauert dementsprechend in der Regel 2 bis 20 h, insbesondere 8 bis 12 h.

Bei der an die Trockenmahlung angeschlossenen Lösungsmittelbehandlung kann auf verschiedene Weise vorgegangen werden.

Eine Variante besteht darin, das Mahlgut (nach Abtrennung der Mahlkörper) in einer vorzugsweise konzentrierten Mineralsäure, z.B. in 50 bis 100 gew.-%iger Salzsäure, in 50 bis 100 gew.-%iger Schwefelsäure oder Mischungen dieser Säuren, 2 bis 20 h bei 0 bis 70°C zu quellen und anschließend auf Eiswasser zu fällen. Bevorzugt ist dabei die Quellung in konzentrierter Schwefelsäure (in der Regel 75 bis 79 gew.-%iger Schwefelsäure) bei Raumtemperatur.

Eine weitere Variante ist die analog durchgeführte Quellung in wäßrigen Lösungen anorganischer Basen, wie

Natriumhydroxid, Kaliumhydroxid, Calciumcarbonat und Natriumhydrogencarbonat, oder organischer Basen, wie Methylamin. Das erhaltene Pigment wird hier durch Filtrieren von der wäßrigen Phase abgetrennt.

Schließlich kann das Mahlgut auch in Wasser, in organischen Lösungsmitteln oder wäßrig-organischen Mischungen gequollen werden. Als organische Lösungsmittel eignen dabei insbesondere mit Wasser mischbare Lösungsmittel, wie Dimethylformamid, N-Methylpyrrolidon, Dimethylsulfoxid, Tetrahydrofuran und vor allem Alkylenglykolmonoalkylether, vor allem Ethylenglykolmonobutylether. Die Behandlungstemperaturen liegen hier im allgemeinen bei 5 bis 100°C, die Behandlungsdauern betragen in der Regel 2 bis 16 h.

Die ebenfalls erfindungsgemäßen Pigmentzubereitungen enthalten als wesentliche Komponenten

A) mindestens ein organisches Pigment aus der Klasse der Perylenpigmente und

B) mindestens ein Perylenderivat der Formel I.

Vorzugsweise enthalten die Pigmentzubereitungen zusätzlich C) mindestens ein Kolophoniumharz.

Selbstverständlich können.auch weitere für Pigmentzubereitungen übliche Zusatzstoffe D), z.B. oberflächenaktive Mittel, enthalten sein.

Insbesondere weisen die erfindungsgemäßen Pigmentzubereitungen folgende Zusammensetzung auf:

A) 80 bis 99,98 Gew.-%, bevorzugt 85 bis 89 Gew.-%, des Perylenpigments,

B) 0,02 bis 5 Gew.-%, bevorzugt 0,2 bis 2 Gew.-%, des Perylenderivates I,

C) 0 bis 18 Gew.-%, bevorzugt 8 bis 15 Gew.-%, des Kolophoniumharzes und

D) 0 bis 5 Gew.-% übliche Zusatzstoffe.

Als Perylenpigment kommen dabei alle bekannten Pigmente in Betracht. Neben dem Perylen-3,4,9,10-tetracarbonsäuredianhydrid (C.I. Pigment Red 224) seien beispielhaft das unsubstituierte Perylen-3,4,9,10-tetracarbonsäurediimid (C.I. Pigment Violet 29) und die N,N'-di($C_1$-$C_{18}$-alkyl)-, N,N'-di($C_5$-$C_7$-cycloalkyl)- und N,N'-diarylsubstituierten Diimide (Aryl: insbesondere Phenyl und durch $C_1$-$C_4$-Alkyl oder -Alkoxy oder Phenylazo substituiertes Phenyl) (z.B.: C.I. Pigment Red 123, 149, 178, 179 und 190) genannt.

Die erfindungsgemäßen Pigmentzubereitungen zeichnen sich durch ihre hervorragenden Anwendungseigenschaften, insbesondere ihre koloristischen Eigenschaften, vor allem ihre Farbstärke und Transparenz, und ihre rheologischen Eigenschaften, aus. Die Pigmentteilchen sind weitgehend isometrisch mit einer Teilchengrößenverteilung von in der Regel 50 bis 100 nm.

Sie können vorteilhaft zur Einfärbung von wäßrigen und nichtwäßrigen Systemen eingesetzt werden. Geeignete Anwendungsmedien sind z.B. Kunststoffe, Lacke, Anstrichfarben, Druckfarben und Toner.

Beispiele

A) Herstellung erfindungsgemäßer Perylenderivate I

Beispiel 1

80 g Perylen-3,4,9,10-tetracarbonsäuredianhydrid wurden unter Rühren in einem Gemisch aus 500 g Wasser und 46 g Kaliumhydroxid gelöst. Nach Einstellen eines pH-Wertes von 7,3 durch Zugabe von 50 g 5 gew.-%iger Salzsäure wurde das ausgefällte Kaliumsalz abfiltriert, bei 120°C getrocknet und dann in 800 g Eiswasser mit 80 g Natriumborhydrid versetzt. Nach 48-stündigem Rühren bei 10-15°C wurde die Suspension mit Salzsäure sauer gestellt. Das dabei ausgefällte Produkt wurde abfiltriert, gewaschen und bei 70°C getrocknet.

Es wurden 43 g des Perylenderivates Ib (X = Y = O; $R^3$+$R^4$ =$R^5$+$R^6$ = $R^7$+$R^8$ = =O; $R^9$ = $R^{10}$ = H) erhalten, was einer Ausbeute von 55% entspricht.

Analytische Daten:

Rotes Pulver vom Schmelzpunkt > 300°C;
Elementaranalyse (Gew.-% ber./gef.):

C: 76,2/75,4; H: 2,6/2,8; O: 21,2/21,2;
$^{13}$C-NMR (400 MHz; $D_2SO_4$): $\delta$ = 179,0; 167,7; 165,3; 142,9; 140,9; 140,6; 139,2; 138,6; 135,6; 134,0; 133,2;

129,8; 129,7; 129,6; 129,5, 128,3; 127,1; 126,9; 125,0; 118,2; 117,1; 115,4; 114,2; 78,5 ppm;
IR (KBr): ν = 1760 (s, C=O) , 1715 (s, C=O) $cm^{-1}$.

Beispiel 2

Ein Gemisch aus 150 g Wasser, 30 g des Perylenderivates Ib aus Beispiel 1 und 60 g 40 gew.-%iger wäßriger Methylaminlösung wurde 3 h bei 85°C gerührt und dann mit Salzsäure sauer gestellt. Das dabei ausgefällte Produkt wurde abfiltriert, gewaschen und bei 70°C getrocknet.

Es wurden 24 g des Perylenderivates Id (X = Y = $NCH_3$; $R^3$+$R^4$ = $R^5$+$R^6$ = $R^7$+$R^8$ = =O; $R^9$ = $R^{10}$ = H) erhalten, was einer Ausbeute von 75% entspricht.

Analytische Daten:

Rotes Pulver vom Schmelzpunkt > 300°C;
Elementaranalyse (Gew.-% ber./gef.):
C: 77,2/77,0; H: 3,0/3,1; O: 11,9/12,0; N: 6,9/6,9.

Beispiel 3

Es wurde analog Beispiel 1 vorgegangen, jedoch wurde das zwischenisolierte Kaliumsalz der Perylen-3,4,9,10-tetracarbonsäure 3 h bei 65°C mit 160 g Natriumborhydrid umgesetzt.

Es wurden 62 g des Perylenderivates Ia (X = Y = O; $R^3$ = OH; $R^4$ = H; $R^5$+$R^6$ = $R^7$+$R^8$ = =O; $R^9$ = $R^{10}$ = H) erhalten, was einer Ausbeute von 80% entspricht.

Analytische Daten:

Rotes Pulver vom Schmelzpunkt > 300°C;
Elementaranalyse (Gew.-% ber./gef.):

C: 75,8/74,7; H: 3,2/3,1; 0: 21,1/22,1;
$^{13}$C-NMR (400 MHz; $D_2SO_4$) : δ = 195,5; 166,2; 164,9; 148,5; 146,4; 140,1; 138,2; 137,8; 137,1; 133,1; 133,0; 129,9; 129,6; 129,3; 128,8; 128,3; 127,7; 127,6; 127,5; 125,2; 123,6; 120,2;116,4; 80,0 ppm;
Masse (TOF-SIMS): m/z = 380($M^+$);
IR (KBr): ν = 3600 (s, O-H); 1750 (s, C=O), 1710 (s, C=O), 1325 (s, C-O) $cm^{-1}$.

Beispiel 4

Es wurde analog Beispiel 3 vorgegangen, jedoch wurde das Kaliumsalz der Perylen-3,4,9,10-tetracarbonsäure nicht zwischenisoliert.

Es wurden 54 g des Perylenderivates Ia erhalten, was einer Ausbeute von 70% entspricht.

Beispiel 5

Es wurde analog Beispiel 2 vorgegangen, jedoch wurden 30 g des Perylenderivates Ia aus Beispiel 3 eingesetzt, und die Reaktionszeit betrug 10 h.

Es wurden 32 g eines Gemisches aus dem Perylenderivat Ie (X = Y = $NCH_3$; $R^3$ = OH; $R^4$ = H; $R^5$+$R^6$ = $R^7$+$R^8$ = $R^9$+$R^{10}$ = =O) und Perylen-3,4,9,10-tetracarbonsäuredianhydrid erhalten.

Beispiel 6

Es wurde analog Beispiel 1 vorgegangen, jedoch wurde das zwischenisolierte Kaliumsalz der Perylen-3,4,9,10-tetracarbonsäure 6 h bei 90°C mit 300 g Natriumborhydrid umgesetzt. Das abfiltrierte Produkt wurde zusätzlich mit einer Kaliumcarbonatlösung gewaschen.

Es wurden 31 g des Perylenderivates Ic (X = Y = O; $R^3$ = $R^4$ = H; $R^5$+$R^6$ = $R^7$+$R^8$ = =O; $R^9$ = $R^{10}$ = H) erhalten, was einer Ausbeute von 40% entspricht.

Analytische Daten:

Rotes Pulver vom Schmelzpunkt > 300°C;
$^{13}$C-NMR (400 MHz; $D_2SO_4$) : δ= 166,4; 146,3; 139,2; 129,7; 127,3; 126,7; 131,8; 123,7; 110,2; 70,5 ppm.

Beispiel 7

Es wurde analog Beispiel 2 vorgegangen, jedoch wurden 15 g des Perylenderivates Ic aus Beispiel 6 eingesetzt.
Es wurden 14 g des Perylenderivates If (X = O; Y = $NCH_3$; $R^3$ = $R^4$ = H; $R^5$+$R^6$ = $R^7$+$R^8$ = =O; $R^9$ = $R^{10}$ = H) erhalten, was einer Ausbeute von 90% entspricht.
Analytische Daten:

Rotes Pulver vom Schmelzpunkt > 300°C;
Elementaranalyse (Gew.-% ber./gef.):

C: 79,6/79,5; H: 4,0/3,9; O: 12,7/12,8; N: 3,7/3,8.

Beispiel 8

Ein Gemisch aus 2000 g Wasser, 10 g N-Methylperylen-3,4,9,10-tetracarbonsäuremonoanhydridmonoimid und 4 g Kaliumhydroxid wurde nach Zugabe von 8 g Natriumborhydrid 48 h bei 60°C gerührt. Nach dem Abkühlen wurde die Suspension mit Salzsäure sauer gestellt. Das dabei ausgefällte Produkt wurde abfiltriert, gewaschen und bei 70°C getrocknet.
Es wurden 7 g des Perylenderivates Ih (X = $NC_2H_5$; Y = O; $R^3$ = OH; $R^4$ = H; $R^5$+$R^6$ = $R^7$+$R^8$ = $R^9$+$R^{10}$ = =O) erhalten, was einer Ausbeute von 70% entspricht.
Analytische Daten:

Rotes Pulver vom Schmelzpunkt > 300°C.

Beispiel 9

Ein Gemisch aus 500 g Dimethylsulfoxid, 5 g N,N'-Dimethylperylen-3,4,9,10-tetracarbonsäurediimid und 1500 g SAZ-Perlen (Durchmesser 0,4 mm) wurde in einem Dispermat nach Zugabe von 4,5 g Natriumborhydrid bei 2000Upm 4 h bei 110°C gehalten. Nach Filtration über ein grobes Filter zur Entf ernung der Mahlkörper wurde der pH-Wert des Filtrats mit Salzsäure auf < 1,5 eingestellt. Das dabei ausgefällte Produkt wurde abfiltriert, gewaschen und bei 70°C getrocknet.
Es wurden 4,6 g des Perylenderivates Ie (X = Y = $NCH_3$; $R^3$ = OH; $R^4$ = H; $R^5$+$R^6$ = $R^7$+$R^8$ = $R^9$+$R^{10}$= =O) erhalten, was einer Ausbeute von 92% entspricht.
Analytische Daten:

Rotes Pulver vom Schmelzpunkt > 300°C;
Elementaranalyse (Gew.-% ber./gef.):
C: 74,3/74,3; H: 3,8/3,8; O: 15,2/15,1; N; 6,7/6,8;
$^{13}$C-NMR (400 MHz; $D_2SO_4$): $\delta$= 168,2; 167,4; 166,0; 164,1; 147,7; 142,4; 141,0; 137,9; 137,4; 137,3; 137,1; 132,8; 131,6; 130,8; 130,0; 129,7; 129,0; 128,9; 127,1; 126,7; 123,8; 122,9; 122,3; 121,9; 42,8; 31,5 ppm;
Masse (TOF-SIMS): m/z = 420($M^+$); 403 ($M^+$-OH);
IR (KBr): $\nu$ = 3371 (m, N-H); 3120 (m, Ar-H); 1693, 1656, (s, C=O) $cm^{-1}$.

Beispiel 10

Es wurde analog Beispiel 9 vorgegangen, jedoch wurden 9 g Natriumborhydrid eingesetzt.
Es wurden 4 g des Perylenderivates Ik (X = Y = $NCH_3$; $R^3$ = $R^4$ = H; $R^5$+$R^6$ = $R^7$+$R^8$ = =O; R9 = $R^{10}$ = H) erhalten, was einer Ausbeute von 86% entspricht.
Analytische Daten:

Rotes Pulver vom Schmelzpunkt > 300°C;
$^{13}$C-NMR (400 MHz; $D_2SO_4$): $\delta$ = 164,1; 150,3; 140,1, 139,9; 139,0; 138,5; 132,1; 129,8, 129,1; 126,6; 126,1; 125,9; 112,0; 57,8; 45,3; 35,3 ppm;
Masse (TOF-SIMS) : m/z = 390 ($M^+$) .

Beispiel 11

Ein Gemisch aus 420 g Wasser, 30 g N,N'-Dimethylgerylen-3,4,9,10-tetracarbonsäurediimid und 1 g Kaliumhydroxid wurde nach Zugabe von 27 g Natriumborhydrid 20 h bei 40°C gerührt. Nach dem Abkühlen wurde die Suspension mit Salzsäure sauer gestellt. Das dabei ausgefällte Produkt wurde abfiltriert, gewaschen und bei 70°C getrocknet.

Es wurden 30 g einer Mischung aus Perylen-3,4,9,10-tetracarbonsäuredianhydrid und dem Perylenderivat Ie aus Beispiel 9 erhalten.

Beispiel 12

Ein Gemisch aus 200 g Tetrahydrofuran und 10 g N,N'-Dimethylperylen-3,4,9,10-tetracarbonsäurediimid wurde nach Zugabe von 1 g Lithiumaluminiumhydrid 1 h bei 25°C gerührt. Nach dem Abkühlen wurde die Suspension mit Salzsäure sauer gestellt. Das dabei ausgefällte Produkt wurde abfiltriert, gewaschen und bei 70°C getrocknet.

Es wurden 10 g einer Mischung aus Perylen-3,4,9,10-tetracarbonsäuredianhydrid und dem Perylenderivat Ie erhalten.

Beispiel 13

Ein Gemisch aus 500 g Wasser und 80 g Perylen-3,4,9,10-tetracarbonsäuredianhydrid wurde nach Zugabe von 160 g Natriumborhydrid 3 h bei 70°C gerührt. Nach dem Abkühlen wurde die Suspension mit Salzsäure sauer gestellt. Das dabei ausgefällte Produkt wurde abfiltriert, gewaschen und bei 70°C getrocknet.

Es wurden 77 g des Perylenderivates Ia aus Beispiel 3 erhalten, was einer Ausbeute von 99% entspricht.

Beispiel 14

Ein Gemisch aus 500 g Tetrahydrofuran und 10 g Perylen-3,4,9,10-tetracarbonsäuredianhydrid wurde nach Zugabe von 2,1 g Methylmagnesiumchlorid 10 h unter Rückfluß gerührt. Die Suspension wurde auf Wasser gegeben und dann mit Salzsäure sauer gestellt. Das dabei ausgefällte Produkt wurde abfiltriert, gewaschen und bei 70°C getrocknet.

Es wurden 7,3 g des Perylenderivates Im (X = Y = O; $R^3$+$R^4$ = =$CH_2$; $R^5$+$R^6$ = $R^7$+$R^8$ = $R^9$+$R^{10}$ = =O) erhalten, was einer Ausbeute von 70% entspricht.

Analytische Daten:

Rotes Pulver vom Schmelzpunkt > 300°C.

Beispiel 15

Ein Gemisch aus 100 g Wasser, 10 g des Perylenderivates In aus Beispiel 14 und 60 g 40 gew.-%iger wäßriger Methylaminlösung wurde 3 h bei 85°C gerührt und dann mit Salzsäure sauer gestellt. Das dabei ausgefällte Produkt wurde abfiltriert, gewaschen und bei 70°C getrocknet.

Es wurden 8 g des Perylenderivates In (X = O; Y = $NCH_3$; $R^3$+$R^4$ = =$CH_2$; $R^5$+$R^6$ = $R^7$+$R^8$ = $R^9$+$R^{10}$ = =O) erhalten, was einer Ausbeute von 83% entspricht.

Analytische Daten:

Rotes Pulver vom Schmelzpunkt > 300°C.

B) Herstellung von erfindungsgemäßen Pigmentzubereitungen

Beispiel 16 bis 29

In Beispiel 16 bis 28 wurde ein Gemisch aus 25 g des Perylenrohpigments P, erhalten durch Umsetzung von Perylen-3,4,9,10-tetracarbonsäureanhydrid mit dem entsprechenden primären Amin in wäßrigem Medium unter Druck (vgl. Bsp. 1, DE-A-21 53 087 für C.I. Pigment Red 179), x g des Perylenderivates I und y g eines thermisch polymerisierten Kolophoniumharzes (Dertopol®, Fa. Granell, Frankreich) 10 h (Beispiel 27: 2 h) mit 30 g Achatkugeln (mittlerer Durchmesser etwa 1 cm) in einer Planetenmühle gemahlen.

In Beispiel 23 wurden 25 g des in Beispiel 11 erhaltenen Gemisches aus Perylen-3,4,9,10-tetracarbonsäuredianhydrid und Perylenderivat Ie eingesetzt.

20 g des erhaltenen Mahlguts wurden anschließend in 250 g 76 gew.-%iger Schwefelsäure 16 h bei Raumtemperatur verquollen. Nach Eintrag in 8 1 Eiswasser und 30-minütigem Nachrühren wurde das Produkt abfiltriert, neutral gewaschen und bei 70°C getrocknet.

In Beispiel 29 wurden 25 g C.I. Pigment Red 179 im Gemisch mit Perylenderivat I, Kolophoniumharz und 2 1 Wasser in einer Rührwerkskugelmühle mit 400 g SAZ-Perlen (Durchmesser 0,3-0,4 mm) 2 h bei 1300 Upm gemahlen. Das Mahlgut wurde nach Abtrennung der Mahlkörper ohne zusätzliche Quellung bei 70°C getrocknet.

In allen Fällen wurde eine Pigmentzubereitung erhalten, die in einem Celluloseacetobutyrat-Lacksystem (CAB) einen transparenten, sehr gelbstichig brillanten Rotfarbton ergab.

Im Vergleich dazu ergab ein analog, jedoch nur in Gegenwart des Harzes gemahlenes und anschließend verquollenes C.I. Pigment Red 179 im Lack einen deckenden, blaustichig trüben Rotfarbton.

Weitere Einzelheiten zu diesen Versuchen sind der folgenden Tabelle zu entnehmen.

Tabelle

| Bsp. | Perylenpigment C.I. Pigment | x g | Perylenderivat | y g Harz |
|---|---|---|---|---|
| 16 | Red 179 | 0,25 | Ib | 3,6 |
| 17 | Red 179 | 0,20 | Id | 3,6 |
| 18 | Red 179 | 0,25 | Ia | 3,6 |
| 19 | Red 179 | 0,225 | Ic | 3,6 |
| 20 | Red 179 | 0,25 | If | 3,6 |
| 21 | Red 179 | 0,12 | In | 3,6 |
| 22 | Red 179 | 0,25 | Ie | 3,6 |
| 23 | Red 179 | 0,25 | Mischung aus Bsp. 11 | 3,6 |
| 24 | Red 179 | 0,12 | Ik | 3,6 |
| 25 | Red 179 | 0,25 | Im | 3,6 |
| 26 | Red 179 | 0,15 | In | 3,6 |
| 27 | Red 178 | 0,25 | Ia | - |
| 28 | Red 149 | 0,25 | Ia | - |
| 29 | Red 179 | 0,25 | Ie | 3,6 |

## Patentansprüche

1. Perylenderivate der allgemeinen Formel I

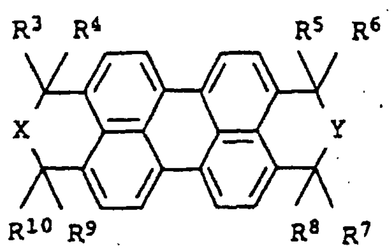

I

in der die Variablen folgende Bedeutung haben:

X und Y unabhängig voneinander Sauerstoff, $-NR^1$ oder $-NR^2$;

$R^1$ und $R^2$ unabhängig voneinander Wasserstoff, $C_1$-$C_{18}$-Alkyl, $C_5$-$C_7$-Cycloalkyl, Aryl oder $C_1$-$C_6$-Alkoxy;

$R^3$ bis $R^{10}$ unabhängig voneinander Wasserstoff, Hydroxy oder Aryl, wobei gemeinsam an ein Kohlenstoffatom gebundene Reste auch =O oder =$CHR^{11}$ bedeuten können;

$R^{11}$ Wasserstoff oder $C_1$-$C_3$-Alkyl,

mit der Maßgabe, daß das Perylenderivat I mindestens eine bis maximal drei Carbonylgruppen pro Molekül enthält.

**2.** Perylenderivate der Formel I nach Anspruch 1, in der $R^5$ und $R^6$ sowie $R^7$ und $R^8$ jeweils zusammen =O bedeuten.

**3.** Perylenderivate der Formel I nach Anspruch 1, in der $R^3$, $R^4$, $R^9$ und $R^{10}$ Wasserstoff oder Hydroxy bedeuten, wobei $R^3$ und $R^4$ oder $R^9$ und $R^{10}$ zusammen auch =O bedeuten können, und $R^5$ und $R^6$ sowie $R^7$ und $R^8$ jeweils zusammen=O bedeuten.

**4.** Verwendung von Perylenderivaten der Formel I gemäß den Ansprüchen 1 bis 3 als Kristallisationsmodifikatoren für organische Pigmente.

**5.** Pigmentzubereitungen, enthaltend

A) mindestens ein organisches Pigment aus der Klasse der Perylenpigmente und

B) mindestens ein Perylenderivat der Formel I gemäß Anspruch 1 sowie gewünschtenfalls

C) mindestens ein Kolophoniumharz.

**6.** Pigmentzubereitungen nach Anspruch 5, enthaltend

A) 80 bis 99,98 Gew.-% des organischen Pigments,

B) 0,02 bis 5 Gew.-% des Perylenderivates der Formel I,

C) 0 bis 18 Gew.-% des Kolophoniumharzes und

D) 0 bis 5 Gew.-% übliche Zusatzstoffe.

## Claims

**1.** Perylene derivatives of the general formula I

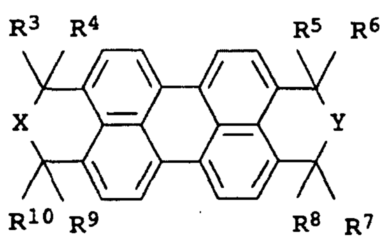

I

where
X and Y are independently oxygen, $-NR^1$ or $-NR^2$;
$R^1$ and $R^2$ are independently hydrogen, $C_1$-$C_{18}$-alkyl, $C_5$-$C_7$-cycloalkyl, aryl or $C_1$-$C_6$-alkoxy;
$R^3$ to $R^{10}$ are independently hydrogen, hydroxyl or aryl, although radicals conjointly attached to one carbon atom may also be =O or =$CHR^{11}$,
$R^{11}$ is hydrogen or $C_1$-$C_3$-alkyl,
with the proviso that said perylene derivative I contains from at least one to not more than 3 carbonyl groups per

molecule.

**2.** Perylene derivatives as claimed in claim 1 of the formula I where the $R^5$ and $R^6$ pair and the $R^7$ and $R^8$ pair are each =O.

**3.** Perylene derivatives as claimed in claim 1 of the formula I where $R^3$, $R^4$, $R^9$ and $R^{10}$ are each hydrogen or hydroxyl, although the $R^3$ and $R^4$ pair or the $R^9$ and $R^{10}$ pair may also be =O, and the $R^5$ and $R^6$ and the $R^7$ and $R^8$ pairs are each =O.

**4.** The use of perylene derivatives of the formula I as set forth in any of claims 1 to 3 as crystallization modifiers for organic pigments.

**5.** Pigment preparations comprising

A) at least one organic pigment from the class of the perylene pigments and

B) at least one perylene derivative of the formula I as set forth in claim 1 and optionally

C) at least one rosin.

**6.** Pigment preparations as claimed in claim 5, comprising

A) from 80 to 99.98% by weight of the organic pigment,

B) from 0.02 to 5% by weight of the perylene derivative of the formula I,

C) from 0 to 18% by weight of the rosin, and

D) from 0 to 5% by weight of customary additives.

**Revendications**

**1.** Dérivés de pérylène de formule générale 1

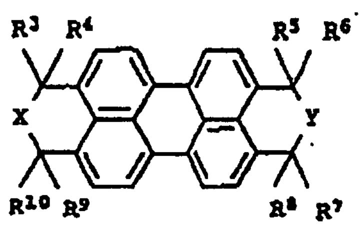

I

dans laquelle les variables ont la signification suivante :

X et Y représentent, indépendamment l'un de l'autre un atome d'oxygène, un groupe $-NR^1$ ou $-NR^2$ ;
$R^1$ et $R^2$, indépendamment l'un de l'autre, représentent un atome d'atome d'hydrogène, un groupe alkyle en $C_1$ à $C_{18}$, cycloalkyle en $C_5$ à $C_7$, aryle ou alcoxy en $C_1$ à $C_6$ ;
$R^3$ à $R^{10}$représentent, indépendamment les uns des autres, un atome d'atome d'hydrogène, un groupe hydroxy ou aryle, les deux groupes liés à un même atome de carbone pouvant également représenter conjointement =O ou $=CHR^{11}$ ;
$R^{11}$ représente un atome d'atome d'hydrogène ou un groupe alkyle en $C_1$ à $C_3$,

à condition que le dérivé de pérylène I contiennent au moins un et jusqu'au maximum 3 groupes carbonyle par molécule.

**2.** Dérivés de pérylène de formule 1 selon la revendication 1, dans lequel $R^5$ et $R^6$ ainsi que $R^7$ et $R^8$ représente conjointement à chaque fois =0.

**3.** Dérivés de pérylène de formule I selon la revendication 1, dans laquelle $R^3$, $R^4$, $R^9$ et $R^{10}$ représente un atome d'atome d'hydrogène ou un groupe hydroxy, $R^3$ et $R^4$ ou $R^9$ et $R^{10}$ pouvant également représenter conjointement =O, et $R^5$ et $R^6$ ainsi que $R^7$ et $R^8$ représentent conjointement à chaque fois =0.

**4.** Utilisation de dérivés de pérylène de formule I selon les revendications 1 à 3 en tant que modificateurs de cristallisation pour des pigments organiques.

**5.** Préparations de pigment, contenant

A) au moins un pigment organique provenant de la classe des pigments de pérylène et
B) au moins un dérivé de pérylène de formule 1 selon la revendication 1 ainsi que, si souhaité
C) au moins une résine colophane.

**6.** Préparations de pigment selon la revendication 5, contenant

A) 80% à 99,98% en poids de pigment organique,
B) 0,02% à 5% en poids du dérivé de pérylène de formule 1,
C) 0% à 18% en poids de la résine colophane et
D) 0% à 5% en poids d'adjuvants habituels.